# FASCICULE DE BREVET EUROPEEN

(11) **EP 1 738 775 B1**
(45) Date de publication et mention de la délivrance du brevet: **29.04.2009**
(21) Numéro de dépôt: 06300518.5
(22) Date de dépôt: 24.05.2006
(51) Int. Cl.: A61L 2/025, A61L 2/02, C02F 1/36, C02F 1/48

(54) **Epurateur bacterien**
Vorrichtung zur Reduzierung der Keimzahl in einem strömenden Medium
Device for reducing the number of bacterial germs in a fluid

(30) Priorité: 26.05.2005 FR 0551385
(43) Date de publication de la demande: 03.01.2007
(73) Titulaire: Drean, Henri, 75016 Paris (FR)
(72) Inventeur: Drean, Henri, 75016 Paris (FR)
(74) Mandataire: Catherine, Alain

(56) Documents cités:
- WO-A-02/34075
- WO-A-97/11908
- WO-A-98/05595
- DE-A1- 10 056 581
- FR-A- 2 841 475
- US-A- 5 965 093

## Description

La présente invention concerne un dispositif destiné au traitement des fluides, en particulier au traitement des eaux ou des gaz, mettant en oeuvre un champ magnétique et des ondes ultrasonores, dans le but de réduire la quantité d'agents pathogènes présents au sein dudit fluide.

La présence, dans l'atmosphère, et/ou les liquides, tels que l'eau, d'une concentration importante en agents pathogènes peut entraîner des nuisances importantes pour les êtres vivants, telles que des maladies.

Parmi les agents pathogènes, potentiellement dangereux pour les êtres vivants, on compte les bactéries, les virus, les champignons, ou encore les moisissures et les levures.

Ces agents pathogènes prolifèrent rapidement en fonction des milieux dans lesquels ils sont présents tels que les liquides ou les aérosols constitués de microgouttelettes de liquide dispersées dans un gaz. Ces agents pathogènes prolifèrent également rapidement en fonction de la température.

Les cellules procaryotes, parmi lesquelles on compte les bactéries, sont de petite taille, généralement de l'ordre de 0,4 µm à 1 µm de diamètre. Ces cellules simples sont limitées par une enveloppe que l'on appelle membrane plasmique comportant un chromosome unique formé d'une molécule d'ADN dans le cytoplasme de la bactérie. La réplication et la transcription du matériel génétique des bactéries s'effectue directement dans le cytoplasme.

Parmi les agents pathogènes qui peuvent être transportés par les gaz et les liquides, on compte également des acaryotes, et notamment des virus. Les virus sont des entités de très petite taille, de l'ordre de 0,001 à 0,1 µm comprenant, à titre de matériel génétique, de courtes séquences d'ADN ou d'ARN, protégées au sein d'une enveloppe protectrice essentiellement protéique, la capside.

La totalité de l'information génétique des agents pathogènes décrits ci-dessus est nécessaire au maintien de leurs fonctions métaboliques.

Compte tenu du danger des agents pathogènes pour l'homme et l'animal, il existe un besoin pour de nouveaux moyens de destruction des agents pathogènes au sein de flux de liquides ou de gaz.

L'un des moyens pour détruire des agents pathogènes est de fragiliser, ou de détruire, les membranes plasmiques ou les capsides, et de manière plus générale les enveloppes de protection du matériel génétique des agents pathogènes.

On connaît du document WO 98/05595 un dispositif destiné au traitement d'un fluide mettant en oeuvre un champ magnétique et des ondes ultrasonores, dans le but de réduire la quantité d'agents pathogènes présents au sein dudit fluide.

L'inventeur a découvert que cette opération de destruction de l'enveloppe de protection du matériel génétique des agents pathogènes, au sein d'un fluide, peut être réalisée en émettant, au sein du fluide, des ondes électromagnétiques et des ondes ultrasonores multiplexées.

En affectant les membranes plasmiques des bactéries par exemple, le cytoplasme ne délimite plus un environnement viable pour la bactérie, et les éléments bactériens deviennent ainsi inopérants. Par ce mécanisme, la bactérie devient non pathogène. Ainsi, les agents pathogènes soumis, au sein du fluide qui les transporte, à des ondes électromagnétiques et à des ondes ultrasonores multiplexées, deviennent des agents non pathogènes, plus aucune fonction biologique ne pouvant être assurée. Ces agents deviennent alors de simples agrégats de macromolécules non pathogènes.

L'inventeur a donc mis au point un dispositif de traitement d'un fluide, destiné à réduire la quantité d'agents pathogènes au sein dudit fluide.

Selon l'invention, ledit dispositif comprend, dans le sens d'écoulement dudit fluide, et selon un axe principal longitudinal :
- un conduit d'entrée du fluide équipé d'un moyen générant un champ électromagnétique au sein dudit conduit d'entrée,
- une enceinte définissant une cavité de traitement dudit fluide et comprenant au moins un moyen de génération d'ondes ultrasonores en direction du centre de ladite cavité,
- un conduit de sortie du fluide, équipé d'un moyen générant un champ électromagnétique au sein dudit conduit de sortie, et
- un réflecteur d'ondes ultrasonores, magnétisable, disposé selon l'axe principal longitudinal dudit dispositif, et comprenant une partie centrale située dans la cavité de l'enceinte et un prolongement situé dans chacun des conduits d'entrée et de sortie du fluide.

Les moyens de génération d'ondes ultrasonores sont disposés autour de l'enceinte de traitement du fluide, de sorte que les ondes ultrasonores émises possèdent un point de convergence commun sur l'axe principal longitudinal.

La présence, au niveau de l'axe principal longitudinal, d'un réflecteur d'ondes ultrasonores, permet une réflexion cylindrique ou « de paroi » des ondes ultrasonores. Ainsi, une concentration d'ondes importante est obtenue au sein de la cavité définie par l'enceinte de traitement. Cette concentration d'ondes importante au sein de l'enceinte de traitement, favorise la destruction des agents pathogènes véhiculés par le flux de fluide traversant l'enceinte de traitement.

De préférence, les conduits d'entrée et de sortie du fluide comprennent des supports qui maintiennent le réflecteur d'ondes ultrasonores selon l'axe principal longitudinal dudit dispositif.

Le réflecteur d'ondes ultrasonores, préférentiellement sous forme de cylindre, est en ferrite de sorte à favoriser la réflexion des ondes vers la partie de l'enceinte de traitement traversée par le fluide.

Par ferrite, on entend un oxyde magnétique, tel que les ferrites de manganèse-zinc ou les ferrites nickel-zinc.

Par « ondes ultrasonores » au sens de la présente invention, on entend, des ondes acoustiques de haute fréquence, dont la gamme d'exploitation se situe entre 10 kHz et 20 MHz, préférentiellement supérieures à 10 kHz, ou mieux encore supérieures à 20 kHz.

A titre d'exemple, dans le domaine des fluides peu visqueux, la gamme d'exploitation des fréquences se situera préférentiellement entre 1 Mhz et 10 MHz, le réglage de la puissance s'effectuant entre 30 et 200 Watt.

Les ondes ultrasonores sont émises à une vitesse de l'ordre de 1480 m/s dans l'eau. Lorsque le flux de fluide traité est de l'eau, la longueur d'onde varie, pour une vitesse de 1480 m/s, de 0,3 mm à 1,5 mm, ce qui correspond respectivement à des fréquences de 5 MHz et 1 MHz.

Les ondes ultrasonores émises ont une direction de préférence perpendiculaire à l'axe principal longitudinal du dispositif de l'invention, donc à la direction d'écoulement du flux de fluide, et des agents pathogènes au sein de celui-ci. Ainsi, la direction du flux de fluide est confondue avec l'axe principal longitudinal du dipositif, et forme un angle d'environ 90° avec la direction des ondes ultrasonores.

Sans vouloir être lié par une quelconque théorie, l'inventeur pense que le dispositif de traitement décrit, favorise la formation d'ondes transversales par rapport à l'axe principal longitudinal, et d'ondes de cisaillement de sorte que la direction de la vibration des agents pathogènes traités au sein du fluide est perpendiculaire à la direction de propagation de l'onde ultrasonore.

Avantageusement, pour éviter les atténuations d'interface, le moyen de génération d'ondes ultrasonores est en contact direct avec le fluide à traiter.

Avantageusement, les moyens de génération d'ondes ultrasonores sont disposés en périphérie de l'enceinte de traitement du fluide, de sorte que les ondes ultrasonores ainsi produites sont émises en direction du réflecteur d'ondes ultrasonores, puis réfléchies par ledit réflecteur.

Ainsi, dans un mode de réalisation particulier de l'invention, le moyen de génération d'ondes ultrasonores, comprenant un émetteur, génère une onde ultrasonore qui traverse le flux de fluide au sein de l'enceinte de traitement, une première fois en direction du réflecteur d'ondes ultrasonores, puis est réfléchie par ce dernier. L'onde traverse ensuite une seconde fois le flux de fluide au sein de l'enceinte de traitement, et revient vers un récepteur à proximité du moyen de génération d'ondes ultrasonores. Un tel récepteur permet d'adapter la puissance et la fréquence d'émission en fonction du parcours de l'onde et de l'efficacité recherchée pour la destruction des agents pathogènes.

De préférence, le dispositif de l'invention comprend au moins deux transducteurs ultrasonores portés par l'enceinte de traitement dans des positions diamétralement opposées.

De préférence, chacun des moyens engendrant un champ électromagnétique, au sein desdits conduits d'entrée et de sortie du fluide, possède une composante de champ magnétique de 1 à 5 Tesla, de préférence, 1 à 2 Tesla.

Avantageusement, chacun des moyens engendrant un champ électromagnétique, au sein desdits conduits d'entrée et de sortie du fluide est une bobine électromagnétique.

De préférence, ledit fluide traversant le dispositif selon l'invention est un gaz ou un liquide, par exemple choisi parmi l'air, la vapeur d'eau ou l'eau.

De préférence, ledit agent pathogène est choisi parmi une bactérie, un virus ou un champignon.

L'invention concerne également un procédé de réduction de la quantité d'agents pathogènes au sein d'un flux de fluide, consistant à soumettre dans une enceinte de traitement dans laquelle le flux s'écoule suivant un axe longitudinal, un flux de fluide à des ondes ultrasonores se propageant de préférence perpendiculairement à l'axe longitudinal d'écoulement du flux, sous l'influence d'un champ magnétique.

La suite de la description se réfère aux figures annexées qui représentent, respectivement :
Figure 1 : une vue générale du dispositif 400 selon l'invention.
Figure 2 : une représentation schématique du dispositif présenté figure 1.
Figure 3 : une vue en coupe du dispositif 400, selon l'axe AA représenté sur la figure 2.
Figure 4 : une représentation en coupe de l'orientation et du cheminement des ondes ultrasonores au sein de l'enceinte de traitement 1.
Figure 5 : une représentation schématique du trajet d'une onde ultrasonore 11 qui se réfléchit sur le réflecteur d'ondes ultrasonores.
Figure 6: un oscillogramme de l'onde 11 citée ci-dessus.
Figure 7 : une représentation spatiale de la propagation des ondes ultrasonores incidentes et réfléchies.

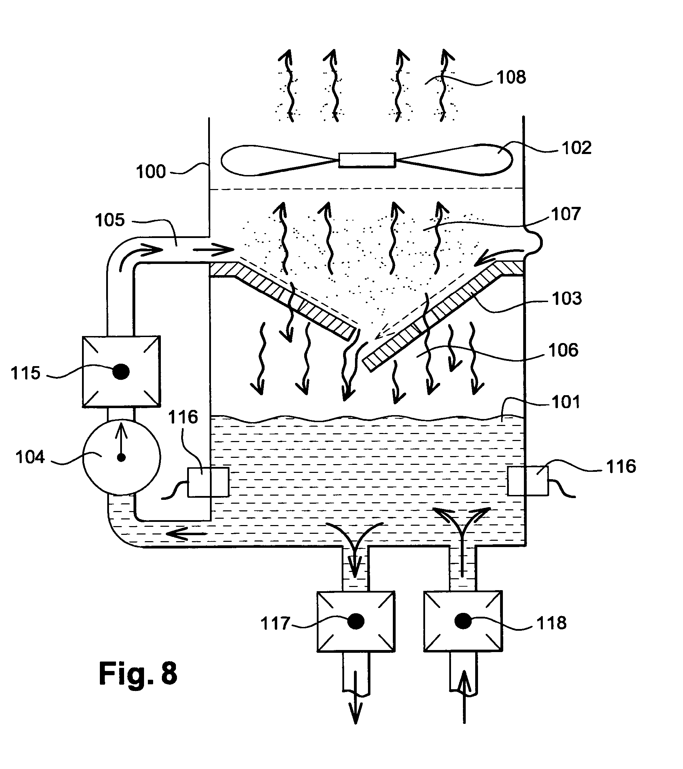

Figure 8 : une tour de réfrigération équipée du dispositif de l'invention.

Dans la description qui suit ainsi que sur les dessins et afin de leur apporter plus de concision et de clarté, les mêmes chiffres de référence sont utilisés pour désigner des organes ou objets identiques permettant la mise en oeuvre du procédé objet de l'invention.

Les parties symétriques du dispositif décrit ci-après, portent les mêmes numéros de référence, auxquels un « prime » est ajouté.

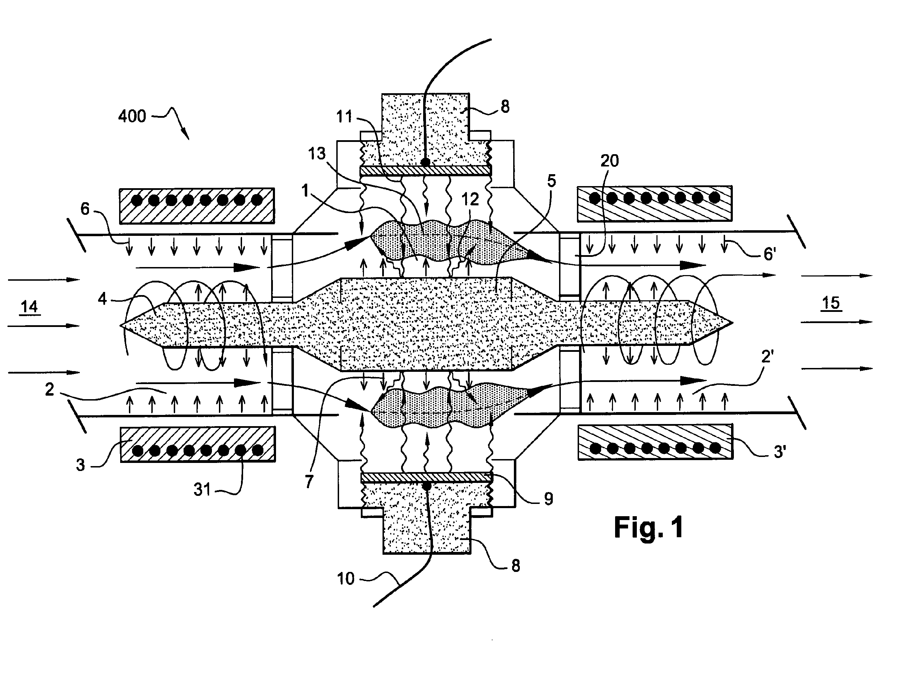

Le dispositif (400) de traitement d'un fluide, représenté sur la figure 1, est destiné à réduire la quantité d'agents pathogènes au sein d'un fluide. Ce dispositif comprend, dans le sens d'écoulement dudit fluide, selon un axe principal longitudinal :
- un conduit d'entrée (2) du fluide équipé d'un moyen (3) générant un champ électromagnétique (6) au sein dudit conduit d'entrée (2),
- une enceinte (1) définissant une cavité de traitement dudit fluide et comprenant au moins un moyen (8) de génération d'ondes ultrasonores en direction du centre de ladite cavité,
- un conduit de sortie (2') du fluide, équipé d'un moyen (3') générant un champ électromagnétique (6') au sein dudit conduit de sortie (2'), et
- un réflecteur d'ondes ultrasonores (4), magnétisable, disposé selon l'axe principal longitudinal dudit dispositif (400), et comprenant une partie centrale (5) située dans la cavité de l'enceinte (1) et un prolongement situé dans chacun des conduits d'entrée (2) et de sortie (2') du fluide.

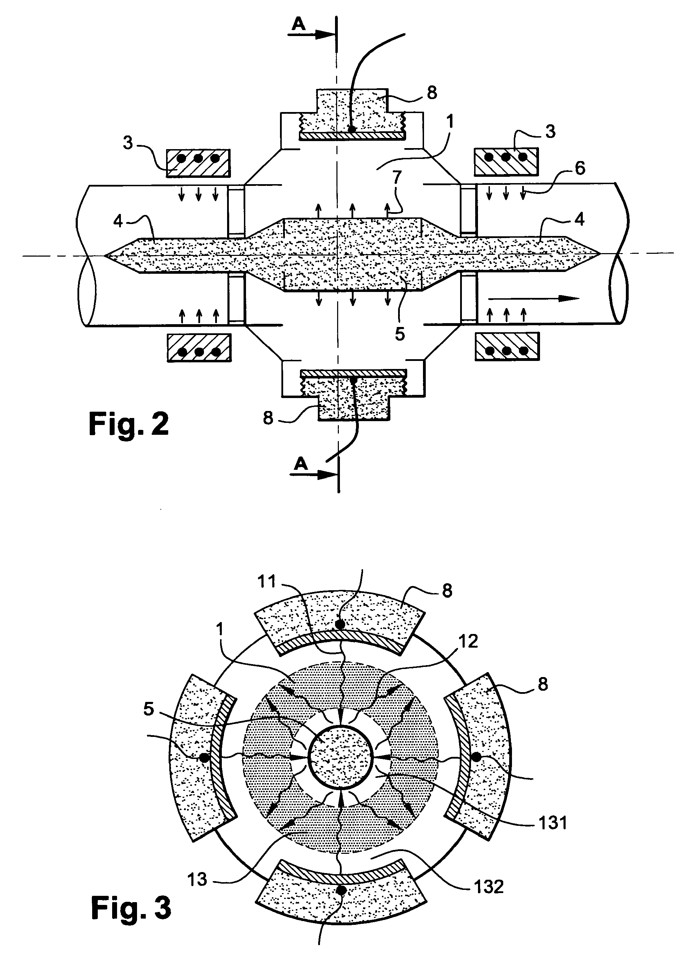

L'enceinte de traitement (1) peut avoir une forme quelconque, mais est de préférence cylindrique comme cela ressortira plus clairement sur la figure 3. Le réflecteur d'ondes ultrasonores (4) comportant de la ferrite magnétique, possède une partie centrale (5), qui devient magnétique sous l'influence des bobines (3) et (3').

Comme on l'observe sur la figure 1, les conduits d'entrée (2) et de sortie (2') du fluide comprennent des supports (20) qui maintiennent le réflecteur d'ondes ultrasonores (4) selon l'axe principal longitudinal du dispositif (400).

chacun des moyens engendrant un champ électromagnétique, de préférence des bobines électromagnétiques, possède une composante de champ magnétique de 1 à 5 Tesla, de préférence, 1 à 2 Tesla.

Ces valeurs permettent tout d'abord d'éviter les dépôts d'un biofilm bactérien sur les parois des conduits d'entrée (2) et de sortie (2') en contact avec le fluide à traiter. Ces valeurs de champ magnétique, permettent également de magnétiser le réflecteur d'ondes ultrasonores (4) de manière directe, et de magnétiser de manière indirecte le centre (5) du réflecteur d'ondes ultrasonores (4). Cette magnétisation du centre (5) du réflecteur (4) permet d'obtenir une induction magnétique forte au niveau de la zone (7) de circulation du fluide dans l'enceinte de traitement (1).

Enfin, de telles valeurs de champ magnétique, permettent de créer une zone propice à la réduction de la quantité d'agents pathogènes au sein dudit fluide et en particulier dans la zone (7) de circulation du fluide.

La présence du réflecteur d'ondes ultrasonores (4) permet d'induire une aimantation permanente. Ainsi, les ondes ultrasonores produites dans l'enceinte de traitement sont accélérées ce qui favorise la destruction des agents pathogènes.

Les bobines (3 et 3') qui génèrent un champ magnétique, comprennent des moyens d'alimentation électrique (31).

Le dispositif (400), tel que représenté sur la figure 1 comprend au moins deux transducteurs ultrasonores (9) portés par l'enceinte de traitement (1) dans des positions diamétralement opposées.

Le ou les moyens de génération d'ondes ultrasonores (8), qui comprennent un transducteur ultrasonore (9), génèrent des ondes ultrasonores qui traversent le flux de fluide au sein de l'enceinte de traitement (1), en direction du réflecteur d'ondes ultrasonores (4), puis ces ondes ultrasonores sont réfléchies par ce dernier. Les ondes ultrasonores traversent ensuite une seconde fois le flux de fluide au sein de l'enceinte de traitement (1) et reviennent vers un récepteur situé à proximité du transducteur (9).

En d'autres termes, les ondes émises en faisceaux (11) traversent la zone de circulation du fluide (7) pour se réfléchir selon l'orientation (12) après réflexion sur le réflecteur d'ondes ultrasonores (4) ; la zone intermédiaire (13) présentant une influence magnétique et des vibrations fortes induites par les ondes ultrasonores. Par conséquent, c'est dans la zone intermédiaire (13) que les agents pathogènes seront détruits.

Comme on l'observe sur les figures 2 et 3, la disposition des transducteurs (8) concentriques permet d'avoir un point de convergence des ondes ultrasonores sur l'axe principal longitudinal du dispositif (400), et plus particulièrement sur le réflecteur d'ondes ultrasonores (4). Le cheminement des ondes ultrasonores émises est représenté par les indices (11) (ondes incidentes), et (12) (ondes réfléchies). Les zones d'influence magnétique centrale (131) et périphérique (132) dans lesquelles les ondes ultrasonores et magnétiques atteignent des vitesses élevées, entraînent la destruction des agents pathogènes et élimine tout risque de formation d'un biofilm.

Sans vouloir être lié à une quelconque théorie, l'inventeur pense que la zone (13) dans laquelle on observe un multiplexage des ondes magnétiques et des ondes ultrasonores, représente la zone essentielle de traitement.

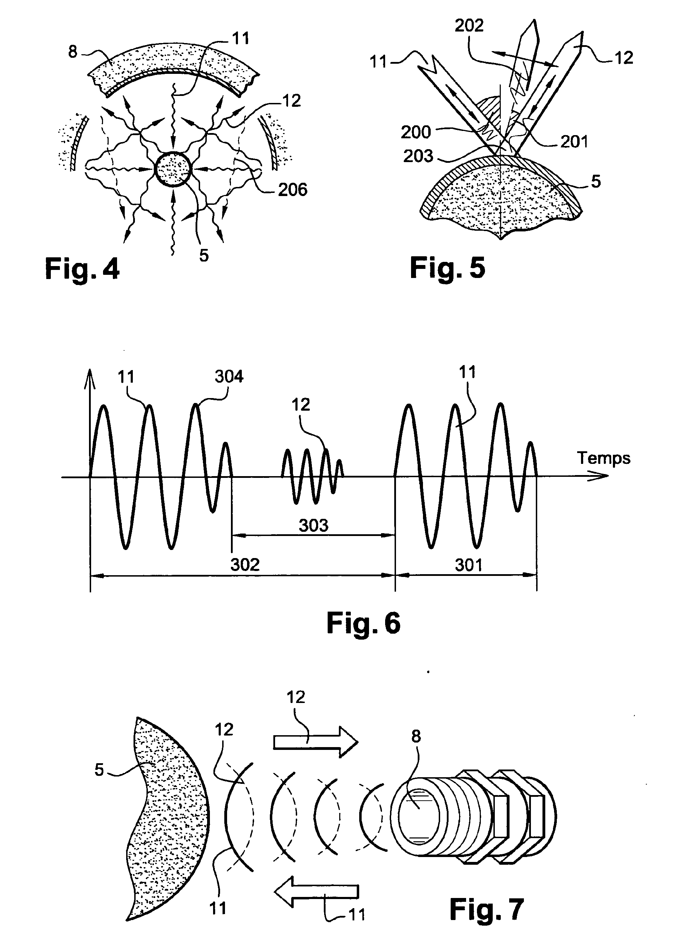

Comme on l'observe sur la figure 4, il peut exister une rétroréflexion des ondes ultrasonores émises (11) et réfléchies (12), sur le cylindre extérieur. Néanmoins, cette rétroréflection d'ondes (206) est atténuée.

Comme cela est illustré sur la figure 5, l'onde longitudinale (11) se réfléchit sur le réflecteur d'ondes ultrasonores (4) avec un angle (200) par rapport à la verticale, sa réflexion se situant en un angle moindre (201) par rapport à une visualisation plane (203). Une onde transversale existe également (202) favorisant le cisaillement en oblique par rapport à l'axe de circulation du fluide.

Sur la figure 6, on observe que l'émission de l'onde (11) représente une amplitude (304) pour une longueur d'onde caractéristique du réglage adopté en fréquence. Le réglage de la puissance et de la fréquence d'émission des ondes ultrasonores s'effectuera en quantifiant les temps d'émission (302) de l'onde réfléchie (303). Le rapprochement de la cadence de l'émission suivante (301) permet de moduler et de multiplexer ces ondes ultrasonores pour une meilleure efficacité du dispositif.

La figure 7 représente la visualisation spatiale de l'enchevêtrement des ondes incidentes (11) et réfléchies (12) et montre l'effet de cisaillement induit par le multiplexage des ondes. Les réglages peuvent être optimisés pour l'émission de l'onde initiale entre 0,1 m/s et 5 m/s en fonction du débit et de la vitesse de circulation du fluide à décontaminer de façon à ce que, pendant le temps de transit dans le dispositif (400), le nombre de trains d'ondes émis se situe entre 10 et 30 ou plus, de sorte à entraîner les vibrations et les ondes de cisaillement nécessaires pour détruire les agents pathogènes contenus dans le fluide à traiter.

Dans un mode de réalisation préféré, le dispositif (400) décrit ci-dessus équipe une tour de réfrigération. Les tours de réfrigération étant connues de l'homme du métier celles-ci ne seront pas décrites en détail ci-après. Brièvement, les tours de réfrigération sont généralement rectangulaires et comportent une enveloppe (100) qui possède à sa partie inférieure un réservoir d'eau (101) qui circule par une pompe (104) pour être injectée en partie haute (105) puis va ruisseler sur des plaques obliques (103) pour effectuer un refroidissement de l'air par mélange. Un flux d'air (107) initié par un ventilateur (102) rejette l'air vers l'atmosphère en (108). Si une contamination bactérienne intervient dans la tour de réfrigération, la contamination bactérienne se retrouvera dispersée par l'humidité relative rejetée à l'extérieur par le flux (108) provoquant ainsi des contaminations autour de la tour de réfrigération. L'utilisation du dispositif (400) sur le recyclage d'injection d'eau en (115) permet de réduire ce risque.

## Revendications

1. Dispositif (400) de traitement d'un fluide, destiné à réduire la quantité d'agents pathogènes au sein dudit fluide, comprenant, dans le sens d'écoulement dudit fluide, selon un axe principal longitudinal:
- un conduit d'entrée (2) du fluide équipé d'un moyen (3) générant un champ électromagnétique (6) au sein dudit conduit d'entrée (2),
- une enceinte (1) définissant une cavité de traitement dudit fluide et comprenant au moins un moyen (8) de génération d'ondes ultrasonores en direction du centre de ladite cavité,
- un conduit de sortie (2') du fluide, équipé d'un moyen (3') générant un champ électromagnétique (6') au sein dudit conduit de sortie (2'), et **caractérisé en ce qu'**il comprend en outre,
- un réflecteur d'ondes ultrasonores (4), magnétisable, disposé selon l'axe principal longitudinal dudit dispositif (400), et comprenant une partie centrale (5) située dans la cavité de l'enceinte (1) et un prolongement situé dans chacun des conduits d'entrée (2) et de sortie (2') du fluide.

2. Dispositif (400) selon la revendication 1, **caractérisé en ce que** ledit réflecteur d'ondes ultrasonores (4) est en ferrite.

3. Dispositif (400) selon la revendication 1 ou 2, **caractérisé en ce que** les conduits d'entrée (2) et de sortie (2') du fluide comprennent des supports (20) qui maintiennent le réflecteur d'ondes ultrasonores (4) selon l'axe principal longitudinal dudit dispositif (400).

4. Dispositif (400) selon l'une quelconque des revendications 1 à 3, **caractérisé en ce que** chacun des moyens engendrant un champ électromagnétique, au sein desdits conduits d'entrée (2) et de sortie (2') du fluide, possède une composante de champ magnétique de 1 à 5 Tesla, de préférence, 1 à 2 Tesla.

5. Dispositif (400) selon l'une quelconque des revendications 1 à 4, **caractérisé en ce que** chacun des moyens engendrant un champ électromagnétique, au sein desdits conduits d'entrée (2) et de sortie (2') du fluide est une bobine électromagnétique.

6. Dispositif (400) selon l'une quelconque des revendications 1 à 5, **caractérisé en ce que** le moyen de génération d'ondes ultrasonores (8) comprend au moins deux transducteurs ultrasonores (9) portés par l'enceinte de traitement (1) dans des positions diamétralement opposées.

7. Utilisation du dispositif (400) selon l'une quelconque des revendications 1 à 6, pour réduire la quantité d'agents pathogènes au sein d'un fluide, **caractérisé en ce que** ledit fluide est un gaz ou un liquide.

8. Utilisation du dispositif (400) selon l'une quelconque des revendications 1 à 6, pour réduire la quantité d'agents pathogènes au sein d'un fluide, **caractérisé en ce que** ledit fluide est choisi parmi l'air, la vapeur d'eau ou l'eau.

9. Utilisation du dispositif (400) selon l'une quelconque des revendications 1 à 6, pour réduire la quantité d'agents pathogènes au sein d'un fluide, **caractérisé en ce que** ledit agent pathogène est choisi parmi une bactérie, un virus ou un champignon.

## Claims

1. Device (400) for treating a fluid, intended to reduce the number of pathogenic agents within said fluid, comprising, in the flowing direction of said fluid, along a longitudinal main axis:
- a fluid inlet duct (2) provided with a means (3) generating an electromagnetic field (6) within said inlet duct (2),
- a chamber (1) defining a cavity for the treatment of said fluid and comprising at least one means (8) for generating ultrasonic waves toward the centre of said cavity,
- a fluid outlet duct (2') provided with a means (3') generating an electromagnetic field (6') within said outlet duct (2'), and **characterized in that** it further comprises:
- a magnetizable ultrasonic-wave reflector (4) arranged along the longitudinal main axis of said device (400) and comprising a central part (5) located in the cavity of the chamber (1) and an extension located in each of the fluid inlet (2) and outlet (2') ducts.

2. Device (400) according to claim 1, **characterized in that** said ultrasonic-wave reflector (4) is made of ferrite.

3. Device (400) according to claim 1 or 2, **characterized in that** the fluid inlet (2) and outlet (2') ducts comprise supports (20) which maintain the ultrasonic-wave reflector (4) along the longitudinal main axis of said device (400).

4. Device (400) according to any one of claims 1-3, **characterized in that** each of the means creating an electromagnetic field within said fluid inlet (2) and outlet (2') ducts has a magnetic-field component of 1-5 Tesla, preferably of 1-2 Tesla.

5. Device (400) according to any one of claims 1-4, **characterized in that** each of the means creating an electromagnetic field within said fluid inlet (2) and outlet (2') ducts is an electromagnetic coil.

6. Device (400) according to any one of claims 1-5, **characterized in that** the means (8) for generating ultrasonic waves comprises at least two ultrasound transducers (9) supported by the treatment chamber (1) at diametrically opposite positions.

7. Use of the device (400) according to any one of claims 1-6 to reduce the number of pathogenic agents within a fluid, **characterized in that** said fluid is a gas or a liquid.

8. Use of the device (400) according to any one of claims 1-6 to reduce the number of pathogenic agents within a fluid, **characterized in that** said fluid is chosen among air, steam or water.

9. Use of the device (400) according to any one of claims 1-6 to reduce the number of pathogenic agents within a fluid, **characterized in that** said pathogen agent is chosen among a bacterium, a virus or a fungus.

## Patentansprüche

1. Vorrichtung (400) zur Behandlung eines Fluids, die zur Reduktion der Menge an Krankheitserregern im Fluid dient und in der Strömungsrichtung des Fluids entlang einer längs verlaufenden Hauptachse Folgendes umfasst:
- eine Einlassleitung (2) für das Fluid, die ein Mittel (3) aufweist, das im Inneren der Einlassleitung (2) ein elektromagnetisches Feld (6) erzeugt,
- eine Ummantelung (1), die einen Behandlungshohlraum für das Fluid definiert und mindestens ein Mittel (8) zur Erzeugung von Ultraschallwellen in Richtung der Mitte des Hohlraums umfasst,
- eine Ausgangsleitung (2') für das Fluid, die ein Mittel (3') aufweist, das im Inneren der Ausgangsleitung (2') ein elektromagnetisches Feld (6') erzeugt,
**dadurch gekennzeichnet, dass** sie ferner Folgendes umfasst:
- einen Ultraschallwellen-Reflektor (4), der magnetisierbar ist, entlang der längs verlaufenden Hauptachse der Vorrichtung (400) angeordnet ist und einen Mittelabschnitt (5), der im Hohlraum der Ummantelung (1) angeordnet ist, sowie eine Verlängerung, die jeweils in der Einlassleitung (2) und in der Auslassleitung (2') für das Fluid angeordnet ist, umfasst.

2. Vorrichtung (400) nach Anspruch 1, **dadurch gekennzeichnet, dass** der Ultraschallwellen-Reflektor (4) aus Ferrit ist.

3. Vorrichtung (400) nach Anspruch 1 oder 2, **dadurch gekennzeichnet, dass** die Einlassleitung (2) und die Auslassleitung (2') für das Fluid Träger (20) umfassen, die den Ultraschallwellen-Reflektor (4) entlang der längs verlaufenden Hauptachse der Vorrichtung (400) halten.

4. Vorrichtung (400) nach einem der Ansprüche 1 bis 3, **dadurch gekennzeichnet, dass** jedes der Mittel, das im Inneren der Einlassleitung (2) und der Auslassleitung (2') für das Fluid ein elektromagnetisches Feld erzeugt, eine magnetische Feldkomponente von 1 bis 5 Tesla, vorzugsweise von 1 bis 2 Tesla, aufweist.

5. Vorrichtung (400) nach einem der Ansprüche 1 bis 4, **dadurch gekennzeichnet, dass** jedes der Mittel, das im Inneren der Einlassleitung (2) und der Auslassleitung (2') für das Fluid ein elektromagnetisches Feld erzeugt, eine elektromagnetische Spule ist.

6. Vorrichtung (400) nach einem der Ansprüche 1 bis 5, **dadurch gekennzeichnet, dass** das Mittel zur Erzeugung von Ultraschallwellen (8) mindestens zwei Ultraschallwandler (9) umfasst, die von der Behandlungsummantelung (1) an diametral entgegengesetzten Positionen getragen sind.

7. Verwendung der Vorrichtung (400) nach einem der Ansprüche 1 bis 6 zur Reduktion der Menge an Krankheitserregern in einem Fluid, **dadurch gekennzeichnet, dass** das Fluid ein Gas oder eine Flüssigkeit ist.

8. Verwendung der Vorrichtung (400) nach einem der Ansprüche 1 bis 6 zur Reduktion der Menge an Krankheitserregern in einem Fluid, **dadurch gekennzeichnet, dass** das Fluid aus Luft, Wasserdampf oder Wasser ausgewählt ist.

9. Verwendung der Vorrichtung (400) nach einem der Ansprüche 1 bis 6 zur Reduktion der Menge an Krankheitserregern in einem Fluid, **dadurch gekennzeichnet, dass** der Krankheitserreger aus einer Bakterie, einem Virus oder einem Pilz ausgewählt ist.
